# EUROPÄISCHE PATENTSCHRIFT

(11) **EP 0 023 343 B1**
(45) Veröffentlichungstag und Bekanntmachung des Hinweises auf die Patenterteilung: **28.03.1984**
(21) Anmeldenummer: 80104315.9
(22) Anmeldetag: 23.07.1980
(51) Int. Cl.: C07D 271/04, C07D 417/12, C07D 295/22, A61K 31/41, A61K 31/455, A61K 31/50, A61K 31/54

(54) **Substituierte 3-Amino-sydnonimine, Verfahren zu ihrer Herstellung und sie enthaltende pharmazeutische Präparate**
Substituted 3-amino-sydnonimines, process for their synthesis and pharmaceutical preparations containing them
3-Amino-sydnonimines substituées, procédé pour leur synthèse et préparations pharmaceutiques les contenant

(30) Priorität: 28.07.1979 DE 2930736
(43) Veröffentlichungstag der Anmeldung: 04.02.1981
(73) Patentinhaber: CASSELLA Aktiengesellschaft, D-60386 Frankfurt (DE)
(72) Erfinder: Schönafinger, Karl, Dr., D-6000 Frankfurt am Main 61 (DE); Beyerle, Rudi, Dr., D-6000 Frankfurt am Main 60 (DE); Nitz, Rolf-Eberhard, Dr., D-6000 Frankfurt am Main 60 (DE); Martorana, Piero A., Dr., D-6380 Bad Homburg v.d.H. (DE); Fiedler, Volker B. Dept. of Pharm. Univ. of Mich., Ann Arbor, Michigan 48109 (US)
(74) Vertreter: Urbach, Hans-Georg

## Beschreibung

Die Erfindung betrifft substituierte 3-Amino-sydnonimine der allgemeinen Formel I 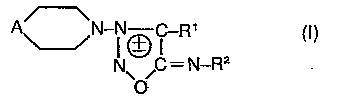 und ihre pharmakologisch annehmbaren Säureadditionssalze, worin
R' Wasserstoff oder Halogen,
R² Wasserstoff, -NO oder eine -COR³⁻ oder -SO₂R⁴-Gruppe,
R³ Wasserstoff, Alkyl mit 1 bis 6 C-Atomen, das auch durch Alkoxi mit 1 bis 6 C-Atomen oder Aryloxi mit 6 bis 12 C-Atomen substituiert sein kann, Cycloalkyl mit 5 bis 8 C-Atomen, Aryl mit 6 bis 12 C-Atomen, das auch durch Halogen und/oder Alkyl mit 1 bis 4 C-Atomen und/oder Alkoxi mit 1 bis 4 C-Atomen mono-, di- oder trisubstituiert sein kann, Aralkyl oder Aralkenyl mit 7 bis 13 C-Atomen, Alkoxi mit 1 bis 6 C-Atomen, Aryloxi mit 6 bis 12 C-Atomen, Pyridyl, Pyrrolyl, Imidazolyl, Pyrazolyl, Pyrazinyl, Pyrimidinyl, Pyridazinyl, Alkoxicarbonyl mit insgesamt 2 bis 7 C-Atomen, A die Gruppe 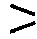 S(O)ₘ, wobei m = 0, 1 oder 2 ist, oder die Gruppe 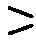 N-SO₂R⁵, R⁴ und R⁵ unabhängig voneinander Alkyl mit 1 bis 6 C-Atomen, Aryl mit 6 bis 12 C-Atomen, das auch durch Methyl oder Chlor substituiert sein kann, oder eine Dialkylaminogruppe mit 1 bis 4 C-Atomen in jedem der Alkylreste bedeuten.

Es sind bereits pharmakologisch wirksame, in 3-Stellung substituierte Sydnonimine (vgl. DE-A1-1942854, DE-A1-1 770 061, FR-A1-2150 306) und ein Verfahren zu ihrer Herstellung (vgl. DE-A1-1 670127) bekannt. Von den bekannten Sydnoniminen befindet sich das 3-Morpholino-N⁶⁻ethoxi- carbonyl-sydnonimin unter der Bezeichnung Molsidomin als Pharmazeutikum zur Heilung und Vorbeugung von cardiovaskulären Erkrankungen im Handel. Gegenüber diesen bekannten Sydnonimi₋ nen sind die erfindungsgemässen Sydnonimine, insbesondere in 3-Stellung, anders substituiert und zeigen dadurch vorteilhaftere pharmakologische Eigenschaften.

Für R' ist Wasserstoff bevorzugt. Für den Fall, dass Rⁱ für Halogen steht, ist Chlor oder Brom bevorzugt. Der für R³ stehende Alkyl-Rest besitzt vorzugsweise 1 bis 4 C-Atome. Falls das für R³ stehende Alkyl durch Alkoxi substituiert ist, besitzen diese Alkoxireste vorzugsweise 1 bis 4 C-Atome. Für R³ stehendes Cycloalkyl wird Cyclohexyl oder Cyclopentyl bevorzugt. Als Arylrest wird für R³ der Phenylrest bevorzugt, der insbesondere durch Chlor, Methyl und/oder Methoxi oder Ethoxi ein-, zwei- oder dreifach substituiert sein kann. Als Arylalkyl-Rest wird für R³ Benzyl und Phenethyl und als Arylalkenyl Styryl bevorzugt. Weitere bevorzugte Reste sind: R² = H, insbesondere, wenn R¹ = H und/oder A = 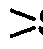 SO₂ ist, sowie R³ = Alkoxi mit 1 oder 2 C-Atomen oder Alkoxicarbonyl mit insgesamt2 oder 3 C-Atomen. Die für R⁴ und/oder R⁵ stehenden Alkylreste sind vorzugsweise Methylreste, die für R⁴ und/oder R⁵ stehenden Arylreste sind insbesondere Phenylreste, die insbesondere auch durch Methyl oder Chlor in 4-Stellung substituiert sein können. Die für R⁴ und/oder R⁵ stehenden Dialkylaminoreste besitzen insbesondere in jeder Alkylgruppe 1 oder 2 C-Atome.

Von den für R³ stehenden heteroaromatischen Pyrrolyl-, Imidazolyl-, Pyrazolyl-, Pyrazinyl-, Pyrimidinyl-, Pyridazinyl- und Pyridyl-Resten ist der Pyridyl-Rest bevorzugt.

Die Verbindungen der allgemeinen Formel I können dadurch hergestellt werden, dass eine Verbindung der allgemeinen Formel II 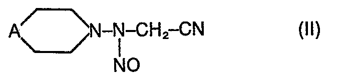 zu einer Verbindung der allgemeinen Formel la 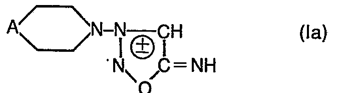 cyclisiert und die so erhaltene Verbindung la, die auch in Form eines Säureadditionssalzes vorliegen kann, mit Acylierungsmitteln, welche den Rest -COR³ oder -SO₂R⁴ einführen, acyliert oder mit salpetriger Säure nitrosiert und/oder zur Einführung von Halogen mit einem Halogen oder einem Halogenierungsmittel umgesetzt und die so erhaltene Verbindung gegebenenfalls in ein Säureadditionssalz überführt wird.

Die Cyclisierung der Verbindung II zu der Verbindung la wird in einem geeigneten anorganischen oder organischen Lösungsmittel, beispielsweise Wasser oder einem Alkanol mit 1 bis 4 C-Atomen, Carbonsäurealkylester, oder einem Gemisch derartiger Lösungsmittel, wie z.B. Wasser-Methanol oder vorzugsweise Essigsäureethylester-Methanol unter Zusatz eines Cyclisierungsmittels, normalerweise bei Temperaturen von 0 bis 40°C, vorzugsweise 0 bis 20°C, durchgeführt. Als Cyclisierungsmittel sind solche geeignet, die in wässriger Lösung einen pH-Wert unter 3 bewirken, also z.B. Mineralsäuren wie Salz-, Schwefel-, Salpeter- oder Phosphorsäure, aber auch starke organische Säuren, wie Trifluoressigsäure. Bei der Cyclisierung wird das entsprechende Säureadditionssalz der Verbindung la erhalten.

Die Verbindungen la stellen erfindungsgemässe Verbindungen für den Fall dar, dass in der allgemeinen Formel I R' = R² = Wasserstoff bedeuten. Zur Einführung der für R² stehenden -NO-Gruppe wird die Verbindung la in an sich bekannter Weise nitrosiert und zur Einführung der für R² stehenden -COR³- oder -SO₂R⁴-Gruppen mit einem geeigneten Acylierungsmittel der allgemeinen Formeln III oder IV
(III) X-COR³ X-SO₂R⁴ (IV), worin X z.B. Halogen, insbesondere Chlor, -OCOR³, O-Aryl, insbesondere Tolyloxi, Nitrophenyloxi oder Dinitrophenyloxi, bedeutet, acyliert. Die Nitrosierung wird in bekannter Weise in einem geeigneten Lösungsmittel, vorzugsweise in Wasser, bei Temperaturen von 0 bis 10°C durchgeführt. Die salpetrige Säure wird dabei normalerweise aus einem Alkalimetallnitrit und Salzsäure erzeugt. Es ist zweckmässig, die wässrige Lösung der Verbindung la mit Salzsäure auf einen pH-Wert von 1 bis 3 einzustellen und das Alkalimetallnitrit in Form einer wässrigen Lösung zu der gerührten und abgekühlten Lösung der Verbindung zuzutropfen. Die Acylierung mit dem Acylierungsmittel III oder IV wird in einem geeigneten Lösungsmittel, wie z. B. Wasser, oder einem polaren organischen Lösungsmittel, wie Dimethylformamid, Dimethylsulfoxid, Pyridin, oder einem Überschuss des Acylierungsmittels, gegebenenfalls in Anwesenheit eines säurebindenden Mittels, wie z.B. Pyridin oder Natriumbicarbonat, unter Rühren bei Temperaturen von 0°C bis zur Siedetemperatur des Lösungs- oder Acylierungsmittels, vorzugsweise von 0 bis 20°C, durchgeführt. Ein für R' stehendes Halogenatom wird durch Umsetzung einer Verbindung la oder vorzugsweise Ib 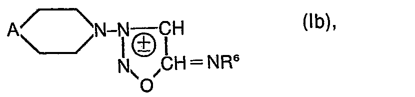 wobei R⁶ -NO, -COR³ oder -SO₂R⁴ bedeutet, mit einem geeigneten Halogenierungsmittel eingeführt. Diese Reaktion wird wie üblich in einem geeigneten Lösungsmittel, wie z.B. einem chlorierten Kohlenwasserstoff oder Eisessig, bei Temperaturen von 0 bis 200C durchgeführt. Als Halogenierungsmittel werden z.B. Halogensuccinimide oder elementares Halogen verwendet. Aus der durch Halogenierung der Verbindung Ib erhaltenen Verbindung kann gewünschtenfalls der für R⁶ stehende Rest -COR³ oder -SO₂R⁴ in an sich bekannter Weise hydrolytisch im sauren Medium abgespalten werden. Man erhält so erfindungsgemässe Verbindungen I mit R' = Halogen und R² = Wasserstoff.

Die substituierten 3-Amino-sydnonimine der allgemeinen Formel 1 bilden mit anorganischen oder organischen Säuren Säureadditionssalze. Solche Säuren sind beispielsweise: Chlorwasserstoff-, Bromwasserstoff-, Phosphor-, Schwefel-, Oxal-, Milch-, Wein-, Essig-, Salicyl-, Benzoe-, Citronen-, Ascorbin-, Adipin- oder Naphthalin-disulfonsäure. Häufig fallen die Säureadditionssalze, insbesondere die Hydrochloride, der Verbindungen der allgemeinen Formel I direkt bei der Synthese der Verbindungen der Formel I an. Aus den Säureadditionssalzen können die freien Verbindungen der allgemeinen Formel 1 gewünschtenfalls in bekannter Weise, d. h. durch Auflösen oder Suspendieren in Wasser und Alkalischstellen, z.B. mit Natronlauge, und anschliessendes Isolieren, gewonnen werden. Pharmakologisch annehmbare Salze werden bevorzugt.

Die benötigten Ausgangsverbindungen der allgemeinen Formel II können nach der Streckerschen Aminonitrilsynthese aus Verbindungen der allgemeinen Formel V 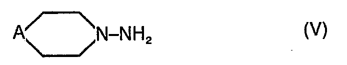 durch Umsetzung mit Formaldehyd und Blausäure in einem geeigneten Lösungsmittel, z.B. Wasser, hergestellt werden, wobei zunächst eine Verbindung der allgemeinen Formel VI 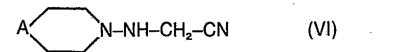 entsteht, die durch Nitrosierung in die Verbindung II überführt wird. Die Nitrosierung wird in bekannter Weise in einem geeigneten Lösungsmittel, vorzugsweise in Wasser, bei Temperaturen von 0 bis 10°C durchgeführt. Die salpetrige Säure wird dabei normalerweise aus einem Alkalimetallnitrit und Salzsäure erzeugt. Es ist zweckmässig, die wässrige Lösung der Verbindung VI mit Salzsäure auf einen pH-Wert von 1 bis 3 einzustellen und das Alkalimetallnitrit in Form einer wässrigen Lösung zu der gerührten und abgekühlten Lösung der Verbindung zuzutropfen.

Die Lösung der so erhaltenen Verbindung II kann direkt der Cyclisierungsreaktion unterworfen werden. Normalerweise ist es jedoch angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel I durchzuführen.

Die Verbindungen der Formel V sind zum Teil bekannt bzw. lassen sich nach der in den Beispielen für die Herstellung von 1-Amino-4-methylsulfonyl-piperazin beschriebenen Methode synthetisieren, bei der das Piperazin VII zunächst mit Kaliumcyanat zu 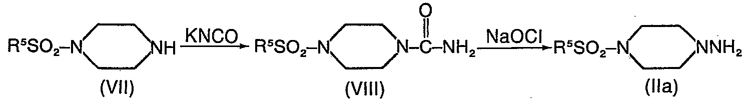 dem Harnstoff VIII und dieser nach dem Hoffmanschen Abbau zu der Verbindung Ila umgesetzt wird.

Erfindungsgemässe Verbindungen der allgemeinen Formel Ic 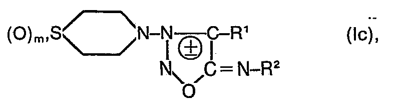 wobei m' = 1 oder 2 ist, lassen sich auch durch Oxidation erfindungsgemässer Verbindungen der allgemeinen Formel Id oder ihrer Säureadditionssalze 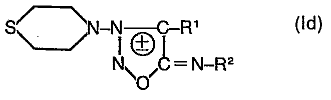 herstellen. Diese Oxidation wird zweckmässigerweise mit Wasserstoffperoxid in einem geeigneten Lösungsmittel, z.B. Eisessig, bei Raumtemperatur oder leicht erhöhter Temperatur (ca. 50 bis 60°C) durchgeführt.

Die Verbindungen der allgemeinen Formel I und ihre pharmakologisch annehmbaren Säureadditionssalze besitzen wertvolle pharmakologische Eigenschaften.

Besonders ausgeprägt ist ihre Wirkung auf das Herz-Kreislauf-System. Verglichen mit der im Handel befindlichen, strukturähnlichen Verbindung Molsidomin oder der ähnlich wirkenden Handelsverbindung Isosorbid-Dinitrat zeigen sie bei einigen Eigenschaften stärkere Wirkungen und/oder wirken über einen längeren Zeitraum. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Die antianginöse Wirkung der erfindungsgemässen Verbindungen kann nach der folgenden Methode gemessen werden.

Die Untersuchungen wurden an Bastardhunden beiderlei Geschlechts in Pentobarbital-Narkose (30 bis 40 mg/kg i. v.) oder in Urethan-Chloralose-Narkose (3 ml/kg Urethan-Chloralose-Gemisch i.v. = 20 mg/kg Chloralose und 250 mg/kg Urethan) durchgeführt. Die Beatmung der Tiere erfolgte mit einem Bird-Mark-7-Respirator. Der endexpiratorische Kohlensäuregehalt (gemessen mit dem Uras) betrug zwischen 4,5 und 5 Vol.-%. Während des gesamten Versuchs erhielten die Tiere mit Pentobarbital-Narkose eine Dauerinfusion von Pentobarbital i.v. = 4 mg/kg/6 ml/h, um eine konstante Narkosetiefe zu gewährleisten; die Tiere mit Urethan-Chloralose-Narkose erhielten keine Dauerinfusion. Die Infusion wurde durch die Vena cephalica gegeben. Nach der Präparation des Versuchstieres wurde ca. 1 Stunde gewartet, bis sich alle haemodynamischen Parameter eingestellt hatten (steady state). Danach wurde mit dem eigentlichen Versuch begonnen.

Der systolische und diastolische Blutdruck wurde peripher in der Arteria femoralis über einen Statham-Druckaufnehmer gemessen. Ein über die Arteria carotis in den linken Ventrikel geschobener Millar-Tip-Katheter lieferte das Signal für den LVEDP (linksventrikulärer enddiastolischer Druck) und die Herzfrequenz. Mit einem zweiten, über die Vena jugularis eingeschobenen Tip-Katheter wurde der mittlere Blutdruck in der Arteria pulmonalis erfasst.

Bei dieser Untersuchungsmethode zeigten beispielsweise die folgenden erfindungsgemässen Verbindungen bereits in den angegebenen Dosierungen eine signifikante Aktivität:
A = 3-(Tetrahydro-1,4-thiazin-4-yl-1,1-dioxid)-sydnoniminhydrochlorid
B = N⁶⁻Cyclohexylcarbonyl-3-(tetrahydro-1,4-thiazin-4-yl-1,1-dioxid)-sydnonimin
C = 3-(4-Methansulfonyl-piperazin-1-yl)-sydnonimin-hydrochlorid
D = N⁶⁻Ethoxicarbonyl-carbonyl-3-(4-methansulfonyl-piperazin-1-yl)-sydnonimin
E = N⁶⁻Benzoyl-3-(4-methansulfonyl-piperazin-1-yl)-sydnonimin
F = 3-(4-Dimethylaminosulfonyl-piperazin-1-yl)-sydnoniminhydrochlorid
G = N⁶⁻(4-Chlorbenzoyl)-3-(tetrahydro-1,4-thiazin-4-yl-1,1-dioxid)-sydnonimin
H = 3-(Thiomorpholino)-sydnonimin-hydrochlorid
I = N⁶⁻Propionyl-3-(4-dimethylaminosulfonyl-piperazin-1-yl)-sydnonimin-hydrochlorid

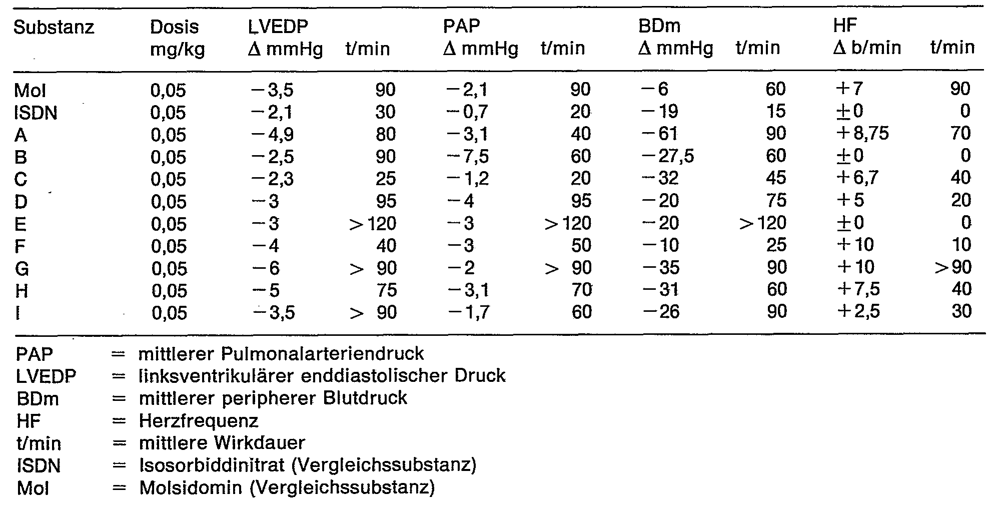

Für die Anwendung in der Therapie werden pharmakologisch annehmbare Säureadditionssalze der neuen Verbindungen oder die neuen Verbindungen in freier Form mit üblichen pharmazeutischen Füll- oder Trägerstoffen, Spreng-, Binde-, Gleit-, Dickungs- oder Verdünnungsmitteln, Lösungsmitteln bzw. Lösungsvermittlern oder Mitteln zur Erzielung eines Depoteffektes gemischt, die eine enterale oder parenterale Anwendung gestatten. Als pharmazeutische Zubereitungsformen kommen z.B. Tabletten, Dragees, Pillen, Kapseln, Lösungen, Suspensionen oder Emulsionen in Frage, wobei ausser den neuen Wirkstoffen noch Konservierungs-, Stabilisierungsmittel, Emulgatoren, Puffersubstanzen und auch noch eine oder mehrere andere therapeutisch wirksame Substanzen zugefügt werden können. Derartige andere therapeutisch wirksame Substanzen sind beispielsweise: ß-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z. B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z. B. Phenprocoumon.

Die pharmazeutischen Zubereitungen enthalten im allgemeinen 0,1 bis 50 mg, vorzugsweise 0,5 bis 10 mg/Dosis.

Die folgenden Beispiele dienen der näheren Erläuterung der Erfindung, wobei Prozentangaben Gewichtsprozente bedeuten, sofern nichts anderes angegeben.

### Beispiel 1

3-Thiomorpholino-sydnonimin-hydrochlorid. 17,7g Aminothiomorpholin werden in 100 g Wasser gelöst. Durch Zutropfen von konzentrierter Salzsäure wird in der Lösung ein pH-Wert von 4 eingestellt; anschliessend wird auf 0-5°C abgekühlt und eine Lösung von 7,4 g Natriumcyanid in 15 g Wasser zugetropft. Nach Zugabe von 12,4 g 40%igem Formalin wird die Lösung über Nacht stehengelassen (pH = 7 bis 7,5). Dann wird mit konzentrierter Salzsäure ein pH-Wert von 2 eingestellt, auf 0 °C abgekühlt und die Lösung von 10,4 g Natriumnitrit in 30 g Wasser langsam zugetropft, 1 Stunde bei 0 bis 5°C nachgerührt, mit zweimal je 50 ml Essigsäureethylester extrahiert und die organische Phase über Natriumsulfat getrocknet. Nach Verdünnen dieser Lösung mit 150 ml Methanol werden unter Wasserkühlung 15 g Chlorwasserstoff eingeleitet und 1 Stunde nachgerührt. Dann wird das feste Produkt abgesaugt, die Mutterlauge eingeengt und aus Ethanol umkristallisiert. Fp.: 181-183°C (Zers.), Ausbeute: 18,5g g (83% der Theorie).

Analog diesem Beispiel lassen sich, ausgehend von
4-Amino-tetrahydro-1,4-thiazin-dioxid,
4-Amino-tetrahydro-1,4-thiazin-oxid,
1-Amino-4-methansulfonyl-piperazin,
1-Amino-4-dimethylamino-sulfonyl-piperazin,
1-Amino-4-p-toluolsulfonyl-piperazin
die folgenden erfindungsgemässen Verbindungen synthetisieren:
3-(Tetrahydro-1,4-thiazin-4-yl-1,1-dioxid)-sydnonimin-hydrochlorid,
Fp.: 206-208 °C (Zers.);
3-(Tetrahydro-1,4-thiazin-4-yl-1-oxid)-sydnonimin-hydrochlorid,
Fp.: 200-201 °C (Zers.);
3-(4-Methansulfonyl-piperazin-1-yl)-sydnonimin-hydrochlorid,
Fp.: 226 °C (Zers.);
3-[4-(p-Toluolsulfonyl)-piperazin-1-yl]-sydnonimin-hydrochlorid,
Fp.: 212°C (Zers.);
3-[4-(Dimethylaminosulfonyl)-piperazin-1-yl]-sydnonimin-hydrochlorid,
Fp.: 193-194 °C (Zers.).

Das als Ausgangsprodukt benötigte 1-Amino-4- methansulfonylpiperazin kann wie folgt synthetisiert werden:
10 g Methansulfonyl-piperazin werden in 50 ml Wasser gelöst; nach der Zugabe von 6,2 ml konzentrierter Salzsäure wird die Lösung von 5,4 g Kaliumcyanat in 20 ml Wasser zugetropft und 4 Stunden bei Raumtemperatur gerührt. Der ausfallende Niederschlag wird abgesaugt und getrocknet. Ausbeute: 11 g, Fp.: 254°C. 10 des getrockneten Niederschlags und 4 g Natronlauge werden in 80 ml Wasser auf 0°C abgekühlt und mit 0,052 Mol auf 0°C vorgekühltem Natriumhypochlorit versetzt. Dann wird bei Raumtemperatur gerührt, bis kein Hypochlorit mehr nachweisbar ist. Diese so erhaltene wässrige Lösung des 1-Aminomethansulfonyl-piperazins kann direkt für die weitere Umsetzung verwendet werden.

Analog können auch andere Ausgangsprodukte der Piperazinreihe hergestellt werden.

### Beispiel 2

### 3-(Tetrahydro-1,4-thiazin-4-yi-1-oxid)sydnonimin-hydrochlorid

17,5 g 3-Thiomorpholino-sydnonimin-hydrochlorid werden in 100 ml Eisessig gelöst. Zu dieser Lösung werden 9,0 g einer wässrigen 30%igen Wasserstoffperoxidlösung zugetropft und eine Stunde bei Zimmertemperatur gerührt. Nach dem Einengen im Vakuum wird aus Ethanol umkristallisiert. Fp.: 200-201 °C (Zers.), Ausbeute: 15,3 g (86% der Theorie).

Analog diesem Beispiel lässt sich durch Verwendung eines grösseren Überschusses an 30%igem Wasserstoffperoxid und durch Verschärfung der Reaktionsbedingungen das 3-(Tetrahydro-1,4-thiazin-4-yl-1,1-dioxid)-sydnonimin-hydrochlorid synthetisieren.

### Beispiel 3

### N⁶⁻Acetyl-3-thlomorpholino-sydnonimin-hydrochlorid.

6,0 g 3-Thiomorpholino-sydnonimin-hydrochlorid werden in 50 mi Acetanhydrid aufgeschlämmt, und nach Zugabe von 10 ml wasserfreiem Pyridin wird über Nacht gerührt. Die farblosen Kristalle werden abgesaugt und aus Methanol umkristallisiert. Fp.: 197°C (Zers.), Ausbeute: 6,3 g (89% der Theorie).

Analog diesem Beispiel lassen sich die folgenden erfindungsgemässen Verbindungen herstellen:
N⁶-Acetyl-3-(tetrahydro-1,4-thiazin-4-yl-1,1-dioxid)-sydnonimin,
Fp.: 212-214°C (Zers.);
N⁶-Methoxiacetyl-3-(tetrahydro-1,4-thiazin-4-yl-1,1-dioxid)-sydnonimin,
Fp.: 188-192 °C, (Zers.);
N⁶-Acetyl-3-(4-dimethylaminosulfonyl-piperazin-1-yl)-sydnonimin-hydrochlorid,
Fp.: 185 °C (Zers.);
N⁶-Acetyl-3-(4-dimethylaminosulfonyl-piperazin-1-yl)-sydnonimin
(Fp.: 176-177 °C (Zers.);
N⁶-Acetyl-3-(4-methansulfonyl-piperazin-1-yl)-sydnonimin-hydrochlorid,
Fp.: 204°C (Zers.);
N⁶⁻Acetyl-3-(4-methansulfonyl-piperazin-1-yl)-sydnonimin,
Fp.: 236°C (Zers.);
N⁶⁻Propionyl-3-(4-dimethylaminosulfonyl- piperazin-1-yl)-sydnonimin-hydrochlorid,
Fp.: 179 °C (Zers.);
N⁶-Formyl-3-(tetrahydro-1,4-thiazin-4-yl-1,1-dioxid)-sydnonimin,
Fp.: 213 °C (Zers.).

### Beispiel 4

### N⁶-Pivaloyl-3-(4-methansulfonyl-piperazin-1-yl)-sydnonimin

28,3 g 3-(4-Methansulfonyl-piperazin-1-yl)-sydnonimin-hydrochlorid werden in 200 ml Wasser gelöst, auf 0°C abgekühlt und unter Umrühren mit 28 g Natriumbicarbonat versetzt. Dann werden 24 g Pivaloylchlorid zugefügt, und die Lösung wird über Nacht bei Raumtemperatur gerührt. Die feste Verbindung wird abgesaugt und aus Ethanol umkristallisiert. Fp.: 94-95°C, Ausbeute. 25,8 g (78% der Theorie).

Durch Auflösen in Methanol und Zugabe methanolischer Salzsäure lässt sich diese Verbindung in das Hydrochlorid vom Fp.: 178°C (Zers.) überführen.

### Beispiel 5

### N⁶-Benzoyl-3-(4-methansulfonyl-piperazin-1-yl)-sydnonimin

5,6 g 3-(4-Methansulfonyl-piperazin-1-yl)-sydnonimin-hydrochlorid
und 2,8 g Benzoylchlorid werden in 50 ml wasserfreiem Pyridin einen Tag lang bei Raumtemperatur gerührt. Der feine, kristalline Niederschlag wird abgesaugt und aus Ethanol umkristallisiert. Fp.: 225°C (Zers.), Ausbeute: 6,0 g (85% der Theorie).

Nach Beispielen 4 und 5 lassen sich die folgenden erfindungsgemässen Verbindungen herstellen:
N⁶-Ethoxicarbonyt-3-(tetrahydro-1,4-thiazin-4-yl-1,1-dioxid)-sydnonimin,
Fp.: 208-210 °C (Zers.);
N⁶-Cyclohexylcarbonyl-3-(tetrahydro-1,4-thiazin-4-yl-1,1-dioxid)-sydnonimin,
Fp.: 160-163 °C;
N⁶-Ethoxicarbonyl-3-thiomorpholino-sydnonimin, Fp.: 166-168 °C;
N⁶-Ethoxicarbonyl-3-(tetrahydro-1,4-thiazin-4-yl-1-oxid)-sydnonimin,
Fp.: 135 °C;
N⁶-(p-Chlorbenzoyl)-3-(tetrahydro-1,4-thiazin-4-yl-1,1-dioxid)-sydnonimin,
Fp.: 248-249 °C (Zers.);
N⁶-Phenoxicarbonyl-3-(tetrahydro-1,4-thiazin-4-yl-1,1-dioxid)-sydnonimin,
Fp.: 213-215 °C (Zers.);
N⁶-Cinnamoyl-3-(4-dimethylaminosulfonyl- piperazin-1-yl)-sydnonimin,
Fp.: 190-192°C;
N⁶⁻Benzoyl-3-(tetrahydro-1,4-thiazin-4-yl-1,1-dioxid)-sydnonimin,
Fp.: 245-247 °C (Zers.);
N⁶-Ethoxicarbonyl-3-[4-(p-toluolsulfonyl)-piperazin-1-yl]-sydnonimin,
Fp.: 190-192 °C;
N⁶-Cyclohexylcarbonyl-3-(4-methansulfonyl-piperazin-1-yl)-sydnonimin,
Fp.: 171-172 °C (Zers.);
N⁶-Phenoxiacetyl-3-[4-(p-toluolsulfonyl)-piperazin-1-yl]-sydnonimin,
Fp.: 150 °C (Zers.);
N⁶⁻Pivaloyl-3-(tetrahydro-1,4-thiazin-4-yl-1,1-dioxid)-sydnonimin,
Fp.: 183 °C (Zers.);
N⁶⁻(3,4,5-Trimethoxibenzoyl)-3-(tetra- hydro-1,4-thiazin-4-yl-1,1-dioxid)-sydnonimin, Fp.: 188 °C;
N⁶⁻Ethoxicarbonyl-3-(4-methansulfonyl-piperazin-1-yl)-sydnonimin,
Fp.: 194 °C (Zers.);
N⁶⁻Ethoxicarbonylcarbonyl-3-(tetrahydro-1,4-thiazin-4-yl-1,1-dioxid)-sydnonimin,
Fp.: 179-181 °C;
N⁶-Neopentylcarbonyl-3-(tetrahydro-1,4-thiazin-4-yl-1,1-dioxid)-sydnonimin,
Fp.: 168-171 °C;
N⁶⁻Ethoxicarbonyl-3-(4-dimethylaminosulfonyl- piperazin-1-yl)-sydnonimin,
Fp.: 167-168 °C;
N⁶⁻Nicotinoyl-3-(4-methansulfonyl-piperazin-1-yl)-sydnonimin,
Fp.: 206-207 °C;
N⁶⁻Nicotinoyl-3-(tetrahydro-1,4-thiazin-4-yl-1,1-dioxid)-sydnonimin,
Fp.: 235 °C (Zers.);
N⁶-(2,4-Dichlorbenzoyl)-3-(4-dimethylamino- sulfonyl-piperazin-1-yl)-sydnonimin,
Fp.: 194-196°C;
N⁶-Ethoxicarbonylcarbonyl-3-(4-methansulfonyl-piperazin-1-yl)-sydnonimin,
Fp.: 182 °C (Zers.);
N⁶-Benzoyl-3-(4-dimethylaminosulfonyl-piperazin-1-yl)-sydnonimin,
Fp.: 205-207 °C;
N⁶-Phenylacetyl-3-[4-(p-toluolsulfonyl)-piperazin-1-yl]-sydnonimin,
Fp.: 160°C;
N⁶⁻Butoxiacetyl-3-[4-(p-toluolsulfonyl)-piperazin-1-yl]-sydnonimin,
Fp.: 119-121 °C; N⁶-(p-Toluolsulfonyl)-3-(4-methansulfonyl-piperazin-1-yl)-sydnonimin,
Fp.: 250°C (Zers.);
N⁶⁻(p-Toluolsulfonyl)-3-(tetrahydro-1,4-thiazin-4-yl-1,1-dioxid)-sydnonimin,
Fp.: 167-169 °C.

### Beispiel 6

### N⁶⁻Nitroso-3-(tetrahydro-1,4-thiazin-4-yl-1,1-dioxid)-sydnonimin

5,1 g 3-(Tetrahydro-1,4-thiazin-4-yl-1,1-dioxid)-sydnonimin-hydrochlorid
werden in 30 ml Wasser gelöst. Nach Abkühlen auf 0-5°C wrid die Lösung von 1,4g Natriumnitrit in 15 ml Wasser zugetropft, 1 Stunde bei 5°C nachgerührt und abgesaugt. Fp.: 128-130°C (Zers.), Ausbeute: 3,9 g (78% der Theorie).

### Beispiel 7

### N⁶-Ethoxicarbonyl-3-(4-methansulfonyl-piperazin-1-yl)-4-brom-sydnonimin

3,2 g N⁶⁻Ethoxicarbonyl-3-(4-methansulfonyl-piperazin-1-yl)-sydnonimin
werden in 40 ml Tetrachlorkohlenstoff suspendiert. In kleinen Portionen werden 3,2 g N-Bromsuccinimid eingetragen und das Gemisch anschliessend 30 Minuten auf 50-60 °C erwärmt und kräftig gerührt. Nach dem Abkühlen mit Eiswasser werden die farblosen Kristalle abgesaugt und aus Methanol umkristallisiert. Fp.: 120-122 °C (Zers.), Ausbeute: 3,0 g (63% der Theorie).

Analog diesem Beispiel wird N⁶-Ethoxicarbonyl-3-(tetrahydro-1,4-thiazin-4-yl-1,1-dioxid)-4-brom-sydnonimin vom Fp.: 160-164 °C (Zers.) erhalten.

## Patentansprüche (Patentansprüche für folgende(n) Vertragsstaat(en) : BE, CH, DE, FR, GB, IT, Li, NL, SE)

1. Substituierte 3-Amino-sydnonimine der allgemeinen Formel I 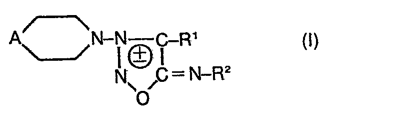 und ihre pharmakologisch annehmbaren Säureadditionssalze, worin
R' Wasserstoff oder Halogen,
R² Wasserstoff, -NO oder eine -COR³⁻ oder -SO₂R⁴-Gruppe,
R³ Wasserstoff, Alkyl mit 1 bis 6 C-Atomen, das auch durch Alkoxi mit 1 bis 6 C-Atomen oder Aryloxi mit 6 bis 12 C-Atomen substituiert sein kann, Cycloalkyl mit 5 bis 8 C-Atomen, Aryl mit 6 bis 12 C-Atomen, das auch durch Halogen und/oder Alkyl mit 1 bis 4 C-Atomen und/oder Alkoxi mit 1 bis 4 C-Atomen mono-, di- oder trisubstituiert sein kann, Aralkyl oder Aralkenyl mit 7 bis 13 C-Atomen, Alkoxi mit 1 bis 6 C-Atomen, Aryloxi mit 6 bis 12 C-Atomen, Pyridyl, Pyrrolyl, Imidazolyl, Pyrazolyl, Pyrazinyl, Pyrimidinyl, Pyridazinyl, Alkoxicarbonyl mit insgesamt 2 bis 7 C-Atomen, A die Gruppe 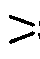 S(O)ₘ, wobei m = 0, 1 oder 2 ist, oder die Gruppe 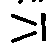 N-SO₂R⁵, R⁴ und R⁵ unabhängig voneinander Alkyl mit 1 bis 6 C-Atomen, Aryl mit 6 bis 12 C-Atomen, das auch durch Methyl oder Chlor substituiert sein kann, oder eine Dialkylaminogruppe mit 1 bis 4 C-Atomen in jedem derAlkylreste bedeuten.

2. 3-Amino-sydnonimine nach Anspruch 1, dadurch gekennzeichnet, dass R' Wasserstoff bedeutet.

3. 3-Amino-sydnonimine nach dem Anspruch 1 oder 2, dadurch gekennzeichnet, dass R² eine -COR³-Gruppe und R³ Alkyl mit 1 bis 4 C-Atomen bedeuten.

4. 3-Amino-sydnonimine nach dem Anspruch 1 oder 2, dadurch gekennzeichnet, dass R² Wasserstoff bedeutet.

5. 3-Amino-sydnonimine nach einem oder mehreren der Ansprüche 1, 2 oder 4, dadurch gekennzeichnet, dass R' und R² Wasserstoff bedeuten.

6. 3-Amino-sydnonimine nach einem oder mehreren der Ansprüche 1 bis 5, dadurch gekennzeichnet, dass A den Rest 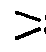 SO₂ bedeutet.

7. 3-Amino-sydnonimine nach einem oder mehreren der Ansprüche 1, 2 oder 6, dadurch gekennzeichnet, dass R² eine -COR3-Gruppe und R³ Pyridyl bedeuten.

8. 3-Amino-sydnonimine nach einem oder mehreren der Ansprüche 1, 2 oder 6, dadurch gekennzeichnet, dass R² eine -COR³-Gruppe und R³ Alkoxi mit 1 oder 2 C-Atomen bedeuten.

9. 3-Amino-sydnonimine nach einem oder mehreren der Ansprüche 1, 2 oder 6, dadurch gekennzeichnet, dass R² eine -COR³-Gruppe und R³ einen Alkoxicarbonylrest mit insgesamt 2 oder 3 C-Atomen bedeuten.

10. 3-Amino-sydnonimine nach Anspruch 1, dadurch gekennzeichnet, dass R¹=R²=Wasserstoff und A = 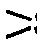 SO₂ bedeuten.

11. Verfahren zur Herstellung der Verbindungen der allgemeinen Formel I des Anspruchs 1, dadurch gekennzeichnet, dass eine Verbindung der allgemeinen Formel II 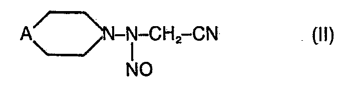 zu einer Verbindung der allgemeinen Formel la, die auch in Form eines Säureadditionssalzes vorliegen kann, 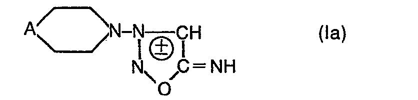 cyclisiert wird und die so erhaltene Verbindung la oder ihr Säureadditionssalz gegebenenfalls a) mit Acylierungsmitteln, welche den Rest -COR³ oder -SO₂R⁴ einführen, acyliert oder nitrosiert und/ oder gegebenenfalls b) mit Halogen oder Halogenierungsmitteln halogeniert wird, wobei die gegebenenfalls erforderlichen Schritte a) und b) in beliebiger Reihenfolge durchgeführt werden, und die so erhaltene Verbindung gegebenenfalls in das Säureadditionssalz überführt wird.

12. Verfahren nach Anspruch 11, dadurch gekennzeichnet, dass als Acylierungsmittel eine Verbindung der Formel XCOR³ oder XSO₂R⁴, worin X OCOR³ oder Halogen, insbesondere Chlor, -0-Aryl, insbesondere Tolyloxi, Nitrophenyloxi, Dinitrophenyloxi, bedeutet, verwendet und die Acylierung ohne Lösungsmittel oder in einem Lösungsmittel, gegebenenfalls in Anwesenheit eines säurebindenden Mittels durchgeführt wird.

13. Pharmazeutisches Präparat, dadurch gekennzeichnet, dass es eine Verbindung der allgemeinen Formel I des Anspruchs 1 oder ein Säureadditionssalz davon als Wirkstoff enthält.

14. Pharmazeutisches Präparat nach Anspruch 13, dadurch gekennzeichnet, dass es noch eine oder mehrere andere pharmazeutisch wirksame Substanzen enthält.

## Patentansprüche (Patentansprüche für folgende(n) Vertragsstaat(en) : AT)

1. Verfahren zur Herstellung substituierter 3-Amino-sydnonimine der allgemeinen Formel I 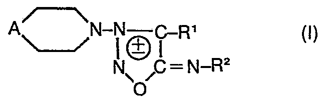 und ihrer pharmakologisch annehmbaren Säureadditionssalze, worin
R' Wasserstoff oder Halogen,
R² Wasserstoff, -NO oder eine -COR³⁻ oder -SO₂R⁴-Gruppe,
R³ Wasserstoff, Alkyl mit 1 bis 6 C-Atomen, das auch durch Alkoxi mit 1 bis 6 C-Atomen oder Aryloxi mit 6 bis 12 C-Atomen substituiert sein kann, Cycloalkyl mit 5 bis 8 C-Atomen, Aryl mit 6 bis 12 C-Atomen, das auch durch Halogen und/oder Alkyl mit 1 bis 4 C-Atomen und/oder Alkoxi mit 1 bis 4 C-Atomen mono-, di- oder trisubstituiert sein kann, Aralkyl oder Aralkenyl mit 7 bis 13 C-Atomen, Alkoxi mit 1 bis 6 C-Atomen, Aryloxi mit 6 bis 12 C-Atomen, Pyridyl, Pyrrolyl, Imidazolyl, Pyrazolyl, Pyrazinyl, Pyrimidinyl, Pyridazinyl, Alkoxicarbonyl mit insgesamt 2 bis 7 C-Atomen, A die Gruppe 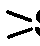 S(O)ₘ, wobei m = 0, 1 oder 2 ist, oder die Gruppe 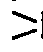 N-SO₂R⁵, R⁴ und R⁵ unabhängig voneinander Alkyl mit 1 bis 6 C-Atomen, Aryl mit 6 bis 12 C-Atomen, das auch durch Methyl oder Chlor substituiert sein kann, oder eine Dialkylaminogruppe mit 1 bis 4 C-Atomen in jedem der Alkylreste bedeuten, dadurch gekennzeichnet, dass eine Verbindung der allgemeinen Formel 11 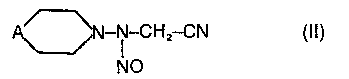 zu einer Verbindung der allgemeinen Formel la, die auch in Form eines Säureadditionssalzes vorliegen kann, 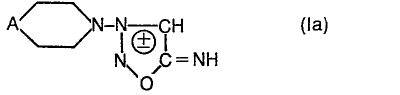 cyclisiert wird und die so erhaltene Verbindung la oder ihr Säureadditionssalz gegebenenfalls a) mit Acylierungsmitteln, welche den Rest -COR³ oder -SO_{Z}R⁴ einführen, acyliert oder nitrosiert und/ oder gegebenenfalls b) mit Halogen oder Halogenierungsmitteln halogeniert wird; wobei die gegebenenfalls erforderlichen Schritte a) und b) in beliebiger Reihenfolge durchgeführt werden, und die so erhaltene Verbindung gegebenenfalls in das Säureadditionssalz überführt wird.

2. Verfahren nach Anspruch 1, dadurch gekennzeichnet, dass die Cyclisierung in einem Lösungsmittel mit Hilfe einer Mineralsäure oder starken organischen Säure bei Temperaturen von 0 bis 40 °C, vorzugsweise 0 bis 20 °C, durchgeführt wird.

3. Verfahren nach den Ansprüchen 1 und/oder 2, dadurch gekennzeichnet, dass als Acylierungsmittel eine Verbindung der Formel XCOR³ oder XSO_{Z}R", worin X = OCOR³ oder Halogen, insbesondere Chlor, -O-Aryl, insbesondere Tolyloxi, Nitrophenyloxi, Dinitrophenyloxi, bedeutet, verwendet und die Acylierung ohne Lösungsmittel oder in einem Lösungsmittel bei 0°C bis zur Siedetemperatur des Lösungs- oder Acylierungsmittels, vorzugsweise bei 0 bis 20 °C, gegebenenfalls in Anwesenheit eines säurebindenden Mittels, durchgeführt wird.

4. Verfahren nach einem oder mehreren der Ansprüche 1 bis 3, dadurch gekennzeichnet, dass die Halogenierung mit einem Halogensuccinimid oder elementarem Halogen in einem Lösungsmittel bei Temperaturen von 0 bis 20°C durchgeführt und gegebenenfalls anschliessend ein für R⁶ stehender -COR³- oder -SO₂R⁴-Rest in an sich bekannter Weise hydrolytisch abgespalten wird.

5. Verfahren nach einem oder mehreren der Ansprüche 1 bis 4, dadurch gekennzeichnet, dass Nitrosierung in einem Lösungsmittel, vorzugsweise in Wasser, bei 0 bis 10 °C durchgeführt wird.

6. Verfahren nach einem oder mehreren der Ansprüche 1 bis 5, dadurch gekennzeichnet, dass für den Fall, dass A = 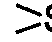 S bedeutet, die erhaltene Verbindung oder ihr Säureadditionssalz zu einer Verbindung mit A = 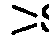 S(O)ₙ, wobei n = 1 oder 2 bedeutet, oxidiert wird, wobei die Oxidation zweckmässigerweise mit Wasserstoffperoxid in einem geeigneten Lösungsmittel durchgeführt wird.

7. Verfahren nach einem oder mehreren der Ansprüche 1 bis 6, dadurch gekennzeichnet, dass die Ausgangsverbindungen so gewählt werden, dass in der Verbindung der allgemeinen Formel I R' Wasserstoff bedeutet.

8. Verfahren nach einem oder mehreren der Ansprüche 1 bis 7, dadurch gekennzeichnet, dass die Ausgangsverbindungen so gewählt werden, dass in der Verbindung der allgemeinen Formel I RZ eine-COR3-Gruppe und R³ Alkyl mit 1 bis 4 C-Atomen bedeuten.

9. Verfahren nach einem oder mehreren der Ansprüche 1 bis 7, dadurch gekennzeichnet, dass die Ausgangsverbindungen so gewählt werden, dass in der Verbindung der allgemeinen Formel I R² Wasserstoff bedeutet.

10. Verfahren nach einem oder mehreren der Ansprüche 1 bis 9, dadurch gekennzeichnet, dass die Ausgangsverbindungen so gewählt werden, dass in der Verbindung der allgemeinen Formel I A den Rest 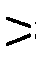 SO₂ bedeutet.

## Claims (Claims for the following Contracting State(s) : s: BE, CH, DE, FR, GB, IT, LI, NL, SE)

1. Substituted 3-amino-sydnone-imines of the general formula I 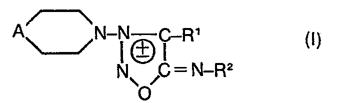 and its pharmacologically acceptable acid addition salts, wherein R' denotes hydrogen or halogen, R² denotes hydrogen, -NO or a -COR³⁻ or -SO₂R⁴-group, R³ denotes hydrogen, alkyl with 1 to 6 atoms, which can also be substituted by alkoxy with 1 to 6 C-atoms or aryloxy with 6 to 12 C-atoms, cycloalkyl with 5 to 8 C-atoms, aryl with 6 to 12 C-atoms, which can also be mono-, di- or trisubstituted by halogen and/or alkyl with 1 to 4 C-atoms and/or alkoxy with 1 to 4 C-atoms, aralkyl with 7 to 13 C-atoms, alkoxy with 1 to 6 C-atoms, aryloxy with 6 to 12 C-atoms, pyridyl, pyrrolyl, imidazolyl, pyrazolyl, pirazinyl, pyrimidinyl, pyridazinyl, alkoxycarbonyl with a total of 2 to 7 C-atoms, A denotes the group 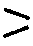 S(O)ₘ, in which m is 0, 1 or 2, or the group 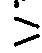 N-SO₂R⁵, R⁴ and R⁵, independently of one another denote alkyl with 1 to 6 C-atoms, aryl with 6 to 12 C-atoms, which can also be substituted by methyl or chlorine, or a dialkylamino group with 1 to 4 C-atoms in each of the alkyl radicals.

2. 3-Amino-sydnone-imines according to Claim 1, characterised in that R' denotes hydrogen.

3. 3-Amino-sydnone-imines according to Claim 1 or 2, characterised in that R² denotes a -COR³- group and R³ denotes alkyl with 1 to 4 C-atoms.

4. 3-Amino-sydnone-imines according to Claim 1 or 2, characterised in that R² denotes hydrogen.

5. 3-Amino-sydnone-imines according to one or several of Claims 1, 2 or 4, characterised in that R' and R² denote hydrogen.

6. 3-Amino-sydnone-imines according to one or several of Claims 1 to 5, characterised in that A denotes the radical 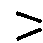 SO₂.

7. 3-Amino-sydnone-imines according to one or several of Claims 1, 2 or 6, characterised in that R² denotes a -COR³-group and R³ denotes pyridyl.

8. 3-Amino-sydnone-imines according to one or several of Claims 1, 2 or 6, characterised in that R² denotes a -COR³-group and R³ denotes alkoxy with 1 or 2 C-atoms.

9. 3-Amino-sydnone-imines according to one or several of Claims 1, 2 or 6, characterised in that R² denotes a -COR³-group and R³ denotes an alkoxycarbonyl radical with a total of 2 or 3 C-atoms.

10. 3-Amino-sydnone-imines according to Claim 1, characterised in that R' = R² = hydrogen and A is 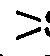 SO₂.

11. Process for the preparation of the compounds, of the general formula I, of Claim 1, characterised in that a compound of the general formula II 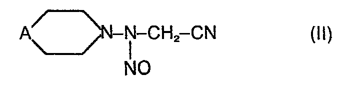 is cyclised to give a compound of the general formula la, which can also be in the form of an acid addition salt, 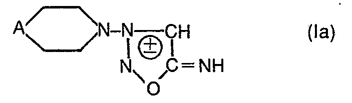 and the resulting compound la, or its acid addition salt, is a) acylated with acylating agents which introduce the radical -COR³ or -SO₂R⁴ or nitrosated and/or, optionally, is b) halogenated with halogen or halogenating agents, the steps a) and b), which may be required, being carried out in any order, and the compound thus obtained is optionally converted to the acid addition salt.

12. Process according to Claim 11, characterised in that the acylating agent used is a compound of the formula XCOR³ or XSOZR3, wherein X is OCOR³ or halogen, in particular chlorine, -0- aryl, in particular tolyloxy, nitrophenyloxy or dinitrophenyloxy, and the acylation is carried out without a solvent or in a solvent, optionally in the presence of an acid-binding agent.

13. Pharmaceutical preparation, characterised in that it contains, as the active compound, a compound of the general formula I, of Claim 1, or an acid addition salt thereof.

14. Pharmaceutical preparation according to Claim 13, characterised in that it also contains one or more further pharmaceutically active substances.

## Claims (Claims for the following Contracting State(s) : Austria)

1. Process for the preparation of substituted 3-amino-sydnone-imines of the general formula I 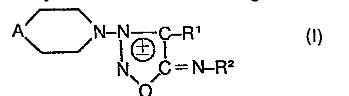 and its pharmacologically acceptable acid addition salts, wherein R' denotes hydrogen or halogen, R² denotes hydrogen, -NO or a -COR³⁻ or -SO_{Z}R⁴-group, R³ denotes hydrogen, alkyl with 1 to 6 atoms, which can also be substituted by alkoxy with 1 to 6 C-atoms or aryloxy with 6 to 12 C-atoms, cycloalkyl with 5 to 8 C-atoms, aryl with 6 to 12 C-atoms, which can also be mono-, di- or trisubstituted by halogen and/or alkyl with 1 to 4 C-atoms and/or alkoxy with 1 to 4 C-atoms, aralkyl with 7 to 13 C-atoms, alkoxy with 1 to 6 C-atoms, aryloxy with 6 to 12 C-atoms, pyridyl, pyrrolyl, imidazolyl, pyrazolyl, pirazinyl, pyrimidinyl, pyridazinyl, alkoxycarbonyl with a total of 2 to 7 C-atoms, A denotes the group 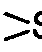 S(O)ₘ, in which m is 0, 1 or 2, or the group 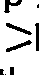 N-SO₂R⁵, R⁴ and R⁵, independently of one another denote alkyl with 1 to 6 C-atoms, aryl with 6 to 12 C-atoms, which can also be substituted by methyl or chlorine, or a dialkylamino-group with 1 to 4 C-atoms in each of the alkyl radicals, characterised in that a compound of the general formula II 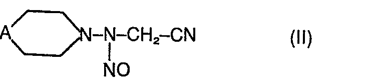 is cyclised to give a compound of the general formula la, which can also be in the form of an acid addition salt, 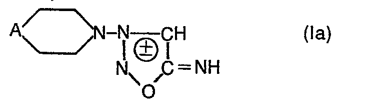 and the resulting compound la, or its acid addition salt, is
a) acylated with acylating agents which introduce the radical COR³ or -O₂R⁴ or nitrosated and/or, optionally, is b) halogenated with halogen or halogenating agents, the steps a) and b), which may be required, being carried out in any order, and the compound thus obtained is optionally converted to the acid addition salt.

2. Process according to Claim 1, characterised in that the cyclisation is carried out in a solvent by means of a mineral acid or strong organic acid at temperatures from 0 to 40 °C, preferably 0 to 20 °C.

3. Process according to Claims 1 and/or 2, characterised in that the acylating agent used is a compound of the formula XCOR³ or XSO₂R⁴, wherein X is OCOR³ or halogen, in particular chlorine, -0-aryl, in particular tolyloxy, nitrophenyloxy or dinitrophenyloxy, and the acylation is carried out without a solvent or in a solvent at 0°C to the boiling temperature of the solvent or acylating agent, preferably at to 20 °C, optionally in the presence of an acid-binding agent.

4. Process according to one or several of Claims 1 to 3, characterised in that the halogenation is carried out with a halogen succinimide or elemental halogen in a solvent at temperatures from 0 to 20°C and, optionally, a -COR³ or -SO₂R⁴ radical representing R⁶ is subsequently hydrolytically split of.

5. Process according to one or several of Claims 1 to 4, characterised in that the nitrosation is carried out in a solvent, preferably in water, at 0 to 10°C.

6. Process according to one or several of Claims 1 to 5, characterised in that, if A denotes 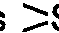 S, the compound obtained or its acid addition salt is oxidised to give a compound A = 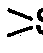 S(O)ₙ, wherein n denotes 1 or 2, the oxidation being appropriately carried out with hydrogen peroxide in a suitable solvent.

7. Process according to one or several of Claims 1 to 6, characterised in that the starting compounds are chosen such that in the compound of general formula I R' denotes hydrogen.

8. Process according to one or several of Claims 1 to 7, characterised in that the starting compounds are chosen such that in the compound of general formula IR2 denotes a -COR³-group and R³ denotes alkyl with 1 to 4 C-atoms.

9. Process according to one or several of Claims 1 to 7, characterised in that the starting compounds are chosen such that in the compound of general formula I R² denotes hydrogen.

10. Process according to one or several of Claims 1 to 9, characterised in that the starting compounds are chosen such that in the compound of general formula I A denotes the radical >SO₂.

## Revendications (Revendications pour l'(les) Etat(s) contractant(s) suivant(s) : BE, DE, FR, GB, IT, LI, NL, SE)

1. Procédé de préparation d'amino-3- sydnonimines substituées de formule générale 1 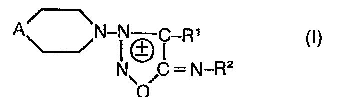 et de leurs sels d'addition acide acceptables d'un point de vue pharmaceutique, où R' est l'hydrogène ou R² est l'hydrogène, -NO ou un radical -COR³ ou -S0₂R⁴, R³ est l'hydrogène, un radical alkyle à 1 à 6 atomes de carbone pouvant aussi être substitué par un radical alcoxy à 1 à 6 atomes de carbone ou aryloxy à 6 à 12 atomes de carbone, cycloalkyle à 5 à 8 atomes de carbone, aryle à 6 à 12 atomes de carbone, pouvant aussi être mono-, bi- ou trisubstitué par un halogène ou un radical alkyle à 1 à 4 atomes de carbone et/ou alcoxy à 1 à 4 atomes de carbone, aralkyle ou aralcényle à 7 à 13 atomes de carbone, alcoxy à 1 à 6 atomes de carbone, aryloxy à 6 à 12 atomes de carbone, pyridyle, pyrrolyle, imidazolyle, pyrazolyle, pyrazinyle, pyrimidinyle, pyridazinyle, alcoxycarbonyle avec en tout 2 à 7 atomes de carbone; A est le radical 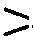 S(O)ₘ où m = 0, 1 ou 2, ou le groupe 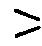 N-SO₂R⁵; R⁴ et R⁵ sont, indépendamment l'un de l'autre, un radical alkyle à 1 à 6 atomes de carbone, aryle à 6 à 12 atomes de carbone pouvant être aussi substitué par un radical méthyle ou le chlore, ou un groupe dialkil- amino à 1 à 4 atomes de carbone dans chacun des radicaux alkyle.

2. Amino-3-sydnonimines selon la revendication 1, caractérisées en ce que R' est l'hydrogène.

3. Amino-3-sydnonimines selon l'une quelconque des revendications 1 ou 2, caractérisées en ce que R² est un radical -COR³ et R³ un radical alkyle à 1 à 4 atomes de carbone.

4. Amino-3-sydnonimines selon l'une quelconque des revendications 1 ou 2, caractérisées en ce que R² est l'hydrogène.

5. Amino-3-sydnonimines selon l'une quelconque des revendications 1, 2 ou 4, caractérisées en ce que R' et R² sont l'hydrogène.

6. Amino-3-sydnonimines selon l'une quelconque des revendications 1 à 5, caractérisées en ce que A est le radical 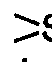 SO₂.

7. Amino-3-sydnonimines selon l'une quelconque des revendications 1, 2 ou 6, caractérisées en ce que R² est un radical -COR³ et R³ un radical pyridyle.

8. Amino-3-sydnonimines selon l'une quelconque des revendications 1, ou 6, caractérisées en ce que R² est un radical -COR³ et R³ un radical alcoxy à 1 ou 2 atomes de carbone.

9. Amino-3-sydnonimines selon l'une quelconque des revendications 1, 2 ou 6, caractérisées en ce que R² est un radical -COR³ et R³ un radical alcoxycarbonyle ayant en tout 2 ou 3 atomes de carbone.

10. Amino-3-sydnonimines selon la revendication 1, caractérisées en ce que R' et R² sont l'hydrogène et A est 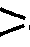 SO₂.

11. Procédé de synthèse des composés de formule générale I selon la revendication 1, caractérisé en ce qu'il consiste à cycliser un composé de formule générale Il 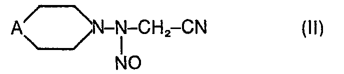 pour le transformer en un composé de formule générale la, qui peut aussi se présenter sous la forme d'un sel d'addition acide 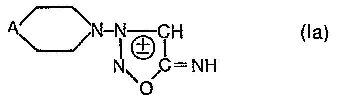 et éventuellement (a) à acyler ou nitroser avec des agents d'acylation qui introduisent le radical -COR³ ou -SO₂R⁴, et/ou éventuellement (b) à halo- géniser à l'aide d'un halogène ou d'halogénation, le composé ainsi obtenu la ou son sel d'addition acide, les étapes (a) et (b) éventuellement nécessaires étant réalisées dans un ordre quelconque, le composé ainsi obtenu étant éventuellement converti en le sel d'addition acide.

12. Procédé selon la revendication 11, caractérisé en ce qu'on utilise en tant qu'agent d'acylation un composé de formule XCOR³ ou XSO_{z}R⁴ où X est OCOR³ ou un halogène, en particulier le chlore, un radical -a-aryle, en particulier le radical tolyloxy, nitrophényloxy, dinitrophényloxy, l'acylation étant réalisée sans solvant ou dans un solvant, éventuellement en présence d'un agent fixateur d'acide.

13. Préparation pharmaceutique, caractérisée en ce qu'elle contient, en tant que substance active, un composé de formule générale I selon la revendication 1 ou un de ses sels d'addition acide.

14. Préparation pharmaceutique selon la revendication 13, caractérisée en ce qu'elle contient encore un ou plusieurs autres principes à activité pharmaceutique.

## Revendications (Revendications pour l'(les) Etat(s) contractant(s) suivant(s) : AT)

1. Procédé de préparation d'amino-3- sydnonimines substituées de formule générale 1 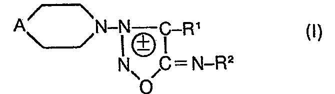 et de leurs sels d'addition acide acceptables d'un point de vue pharmaceutique, où R' est l'hydrogène ou un halogène; R² est l'hydrogène, -NO ou un radical -COR³ ou -SO_{z}R⁴, R³ est l'hydrogène, un radical alkyle à 1 à 6 atomes de carbone pouvant aussi être substitué par un radical alcoxy à 1 à 6 atomes de carbone ou aryloxy à 6 à 12 atomes de carbone, cycloalkyle à 5 à 8 atomes de carbone, aryle à 6 à 12 atomes de carbone, pouvant aussi être mono-, bi- ou trisubstitué par un halogène ou un radical alkyle à 1 à 4 atomes de carbone et/ou alcoxy à 1 à 4 atomes de carbone, aralkyle ou aralcényle à 7 à 13 atomes de carbone, alcoxy à 1 à 6 atomes de carbone, aryloxy à 6 à 12 atomes de carbone, pyridyle, pyrrolyle, imidazolyle, pyrazolyle, pyrazinyle, pyrimidinyle, pyridazinyle, alcoxycarbonyle avec en tout 2 à 7 atomes de carbone; A est le groupe 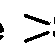 S(O)ₘ où m = 0, 1 ou 2, ou le groupe 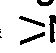 N-SO₂R⁵; R⁴ et R⁵ sont, indépendamment l'un de l'autre, un radical alkyle à 1 à 6 atomes de carbone, aryle à 6 à 12 atomes de carbone pouvant être aussi substitué par un radical méthyle ou le chlore, ou un groupe dialkylamino à 1 à 4 atomes de carbone dans chacun des radicaux alkyle, caractérisé en ce qu'il consiste à cycliser un composé de formule générale Il 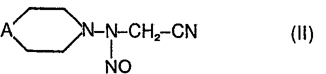 pour le transformer en un composé de formule générale la, qui peut aussi se présenter sous la forme d'un sel d'addition acide 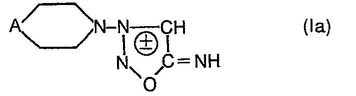 et éventuellement a) à acyler ou nitroser avec des agents d'acylation qui introduisent le radical -COR³ ou -SO₂R⁴, et/ou éventuellement b) à halo- géniser à l'aide d'un halogène ou d'agents d'halogénation, le composé ainsi obtenu la ou son sel d'addition acide, les étapes a) et b) éventùelle- ment nécessaires étant réalisées dans un ordre quelconque, le composé ainsi obtenu étant éventuellement converti en le sel d'addition acide.

2. Procédé selon la revendication 1, caractérisé en ce que la cyclisation s'effectue dans un solvant à l'aide d'un acide minéral ou d'un acide organique fort à des températures de 0 à 40 °C, de préférence de 0 à 20 °C.

3. Procédé selon l'une quelconque des revendications 1 ou 2, caractérisé en ce qu'on utilise en tant qu'agent d'acylation un composé de formule XCOR³ ou XSO₂R⁴ où X estOCOR³ ou un halogène, en particulier le chlore, un radical -O-aryle, en particulier le radical tolyloxy, nitrophényloxy, dinitrophényloxy, l'acylation étant réalisée sans solvant ou dans un solvant à une température comprise entre 0°C et la température d'ébullition du solvant ou de l'agent d'acylation, de préférence entre 0 et 20 °C, éventuellement en présence d'un agent fixateur d'acide.

4. Procédé selon l'une quelconque des revendications 1 à 3, caractérisé en ce que l'halogénation est réalisée à l'aide d'un halogénosuccinimide ou d'halogène élémentaire dans un solvant à des températures de 0 à 20 °C, cette opération étant éventuellement suivie d'une séparation hydrolyti- que, d'une manière connue, d'un radical-COR3 ou -SO₂R⁴ correspondant à R⁶.

5. Procédé selon l'une quelconque des revendications 1 à 4, caractérisé en ce que la nitrosation se déroule dans un solvant, de préférence l'eau, à 0 à 10 °C.

6. Procédé selon l'une quelconque des revendications 1 à 5, caractérisé en ce que, dans le cas où A est 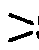 S, le composé obtenu ou son sel d'addition acide est oxydé en un composé avec A = 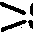 S(O)ₙ où n = 1 ou 2, l'oxydation étant avantageusement réalisée avec du peroxyde d'hydrogène dans un solvant approprié.

7. Procédé selon l'une quelconque des revendications 1 à 6, caractérisé en ce que les composés de départ sont choisis de telle sorte que, dans le composé de formule générale I, R' soit l'hydrogène.

8. Procédé selon l'une quelconque des revendications 1 à 7, caractérisé en ce que les composés de départ sont choisis de telle sorte que, dans le composé de formule générale I, R² soit un groupe -COR³ et R³ un radical alkyle à 1 à 4 atomes de carbone.

9. Procédé selon l'une quelconque des revendications 1 à 7, caractérisé en ce que les composés de départ sont choisis de telle sorte que, dans le composé de formule générale I, R² soit l'hydrogène.

10. Procédé selon l'une quelconque des revendications 1 à 9, caractérisé en ce que les composés de départ sont choisis de telle sorte que, dans le composé de formule générale I, A soit le radical 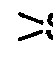 SO₂.
